# EUROPÄISCHE PATENTSCHRIFT

(11) **EP 0 827 549 B1**
(45) Veröffentlichungstag und Bekanntmachung des Hinweises auf die Patenterteilung: **26.09.2001**
(21) Anmeldenummer: 96914170.4
(22) Anmeldetag: 02.05.1996
(51) Int. Cl.: C12P 19/18, C12P 19/12, C12P 19/02, C12P 19/38

(54) **VERFAHREN ZUR ENZYMATISCHEN GALACTOSYLIERUNG VON MONO- UND OLIGOSACCHARIDEN**
METHOD FOR THE ENZYMATIC GALACTOSYLATION OF MONOSACCHARIDES AND OLIGOSACCHARIDES
PROCEDE DE GALACTOSYLATION ENZYMATIQUE DE MONOSACCHARIDES ET D'OLIGOSACCHARIDES

(30) Priorität: 12.05.1995 DE 19516952
(43) Veröffentlichungstag der Anmeldung: 11.03.1998
(73) Patentinhaber: Aventis Research & Technologies GmbH & Co. KG, 65926 Frankfurt am Main (DE)
(72) Erfinder: HÖRSCH, Brigitte, D-65719 Hofheim (DE); SEIFFERT-STÖRIKO, Andreas, D-65929 Frankfurt am Main (DE); MARQUARDT, Rüdiger, D-60389 Frankfurt am Main (DE); ZERVOSEN, Astrid, B-4840 Welkenraedt (BE); ELLING, Lothar, D-52066 Aachen (DE); KULA, Maria, Regina, D-52382 Niederzier (DE)
(74) Vertreter: Ackermann, Joachim, Dr.
(86) Internationale Anmeldenummer: EP9601828
(87) Internationale Veröffentlichungsnummer: WO9635801

(56) Entgegenhaltungen:
- WO-A-94/01540
- GLYCOBIOLOGY, Bd. 3, Nr. 4, 1993, Seiten 349-355, XP002013104 ELLING, L. ET AL.: "Investigation of sucrose synthase from rice for the synthesis of various nucleotide sugars and saccharides" in der Anmeldung erwähnt
- J. ORG. CHEM., Bd. 57, Nr. 16, 1992, Seiten 4343-4344, XP002013105 CHI-HUEY WONG ET AL.: "Regeneration of sugar nucleotide for enzymatic oligosaccharide synthesis"

## Beschreibung

Die Erfindung betrifft ein verbessertes Verfahren zur enzymatischen Galactosylierung von Mono- und Oligosacchariden unter in situ Regeneration des Nucleotidzuckers (bzw. des Nucleosiddiphosphatzuckers) in Gegenwart von Saccharose-Synthase, β-1-4-Galactosyltransferase und Uridindiphosphat-Glucose-4'-Epimerase (UDP-Glucose-4'-Epimerase).

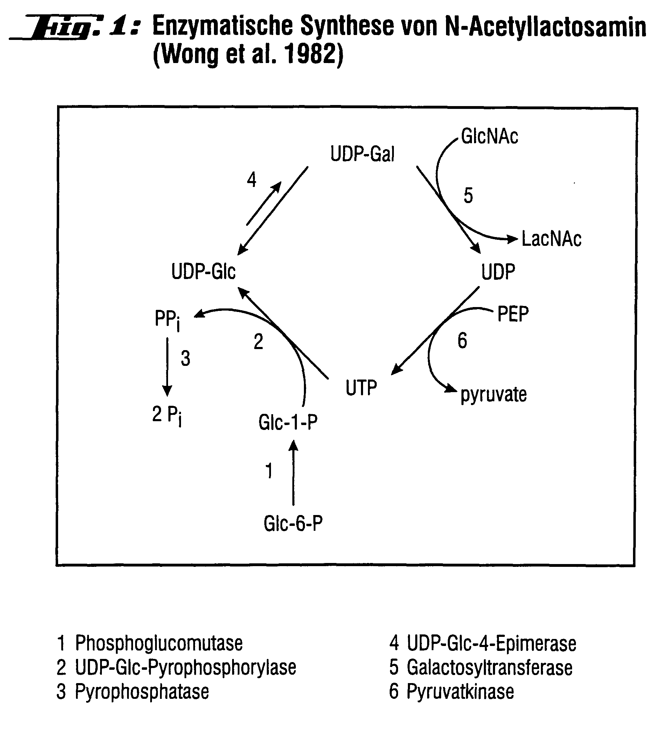

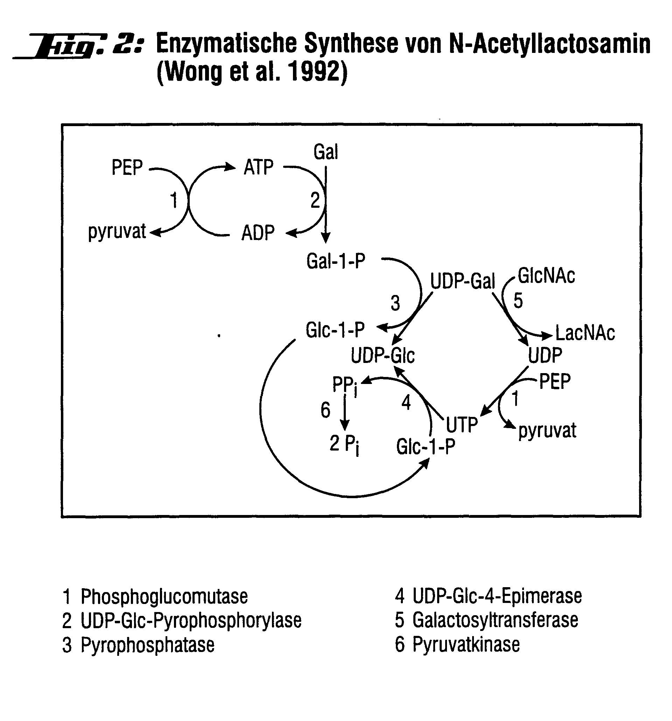

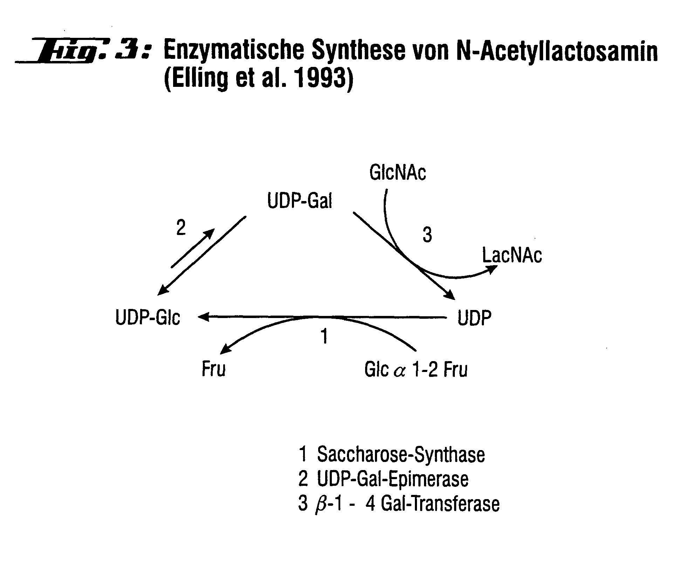

Enzymatische Synthesen von N-Acetyllactosamin (LacNAc) und seinen Derivaten mit der β-1.4 Galactosyltransferase (GaIT) [EC 2.4.1.38] sind bereits seit langem bekannt (C.H. Wong et al.: J. Am. Chem.Soc. 118, 8137 (1991), J. Org.Chem. 57, 4343 (1992)). Wong et al. (Fig. 1 und 2) entwickelten LacNAc-Synthesen mit einer in situ Regeneration von UDP-Glucose, die einen stöchiometrischen Einsatz der teuren Nucleotidzucker überflüssig machte, allerdings 6 verschiedene Enzyme benötigt.

Der von Elling et al. (Glycobiology 3, 349 (1993), DE 42 21 595 C1)) vorgeschlagene LacNAc-Zyklus (Fig. 3) stellt gegenüber den bisher bekannten Zyklen insofern eine Verbesserung dar, daß nur noch drei anstelle von sechs Enzymen zur Synthese eingesetzt werden müssen, die Saccharose-Synthase aus Reis, die β-1-4-Galactosyltransferase und die UDP-Glucose-4'-Epimerase. Die synthetisierten Disaccharide sind Edukte für weitere Umsetzungen mit unterschiedlichen Transferasen, z. B. Sialyltransferasen und Fucosyltransferasen. Zielprodukte dieser enzymatischen Synthesen sind Sialyl Lewis X und seine Derivate (Ichikawa et al. J. Am. Chem. Soc. 114, 9283 (1992)), dessen Bedeutung bei der Zell-Zell-Erkennung Gegenstand einer intensiven Forschung ist (DeFrees et al. J. Am. Chem. Soc. 117, 66 (1995)).

Die Saccharose-Synthase [EC 2.4.1.13] (S. Syn.), eine insbesondere in Pflanzen weit verbreitete Glycosyltransferase, deren Funktion als Katalysator zur Bildung von Nucleotidzuckern innerhalb des Stoffwechsels der Pflanze von Avigad (in Loewus et al. (Eds) Encyclopedia of Plant Physiology New Series Vol. 13A, Carbohydrates I, Intracellular Carbohydrates, Springer Verlag, Berlin, 217 - 347, 1982) zusammenfassend dargestellt wurde, ist zur Synthese von Nucleotidzuckern, wie UDP-, dTDP-, ADP-, CDP- und GDP-Glc, geeignet (Elling et al. Glycobiology 3, 349 (1993)). Die Aufreinigung der Saccharose-Synthase aus Reis und ihr Einsatz zur in situ Regenerierung von UDP-Glucose wurde von Elling et al. (DE 42 21 595 C1, Biotechnol. Appl. Biochem. 21, 29 (1994)) beschrieben. Das Reisenzym ist ein homotetrameres Protein mit einem Molekulargewicht von 362 kDa. Das Enzym wurde bereits zur präparativen Synthese von dTDP-GLc ausgehend von dTDP im Enzymmembranreaktor (EMR) von Zervosen et al. (Angew. Chem. 106, 592 (1994)) eingesetzt.

Zentrales Enzym für die LacNAc- Synthese ist die β-1-4-Galactosyltransferase, die UDP-Galactose auf N-Acetylglucosamin überträgt. Es entsteht N-Acetyllactosamin. Als Acceptoren können eine Reihe weiterer Mono- und Oligosaccharide eingesetzt werden.

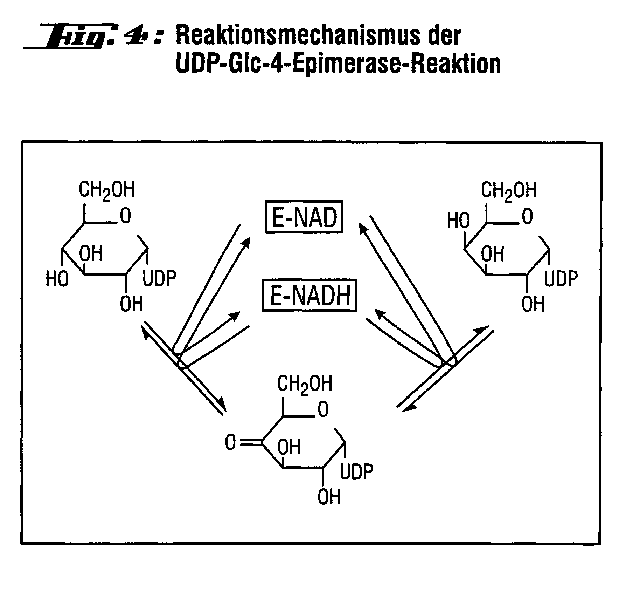

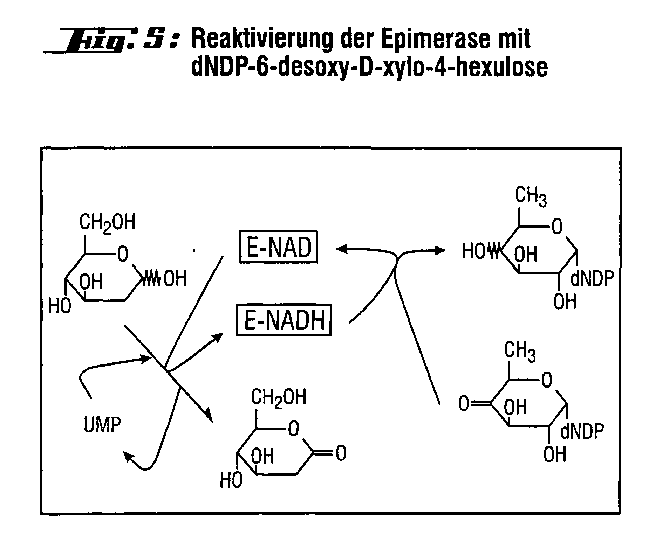

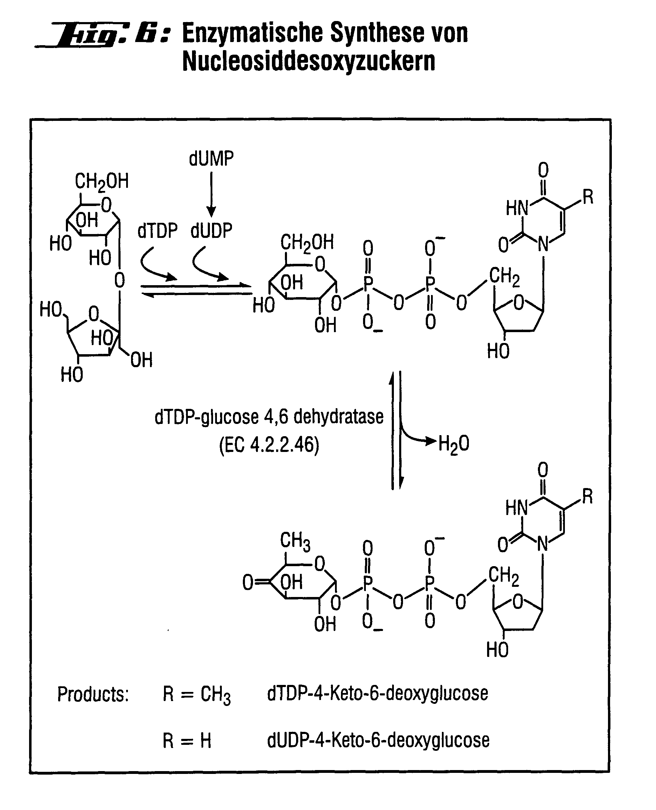

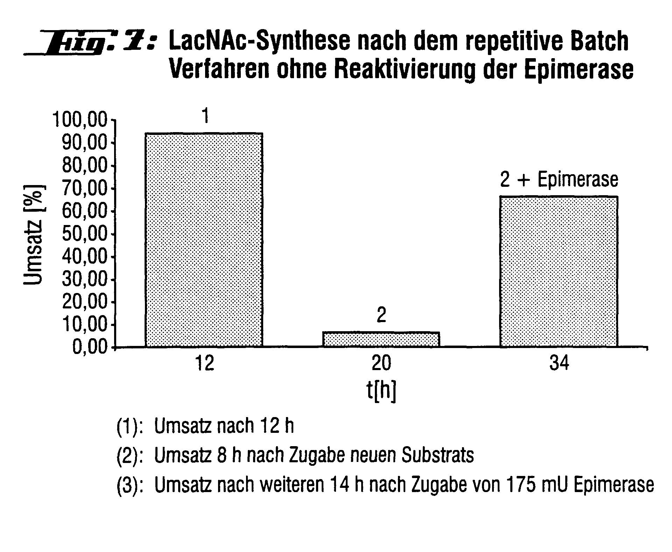

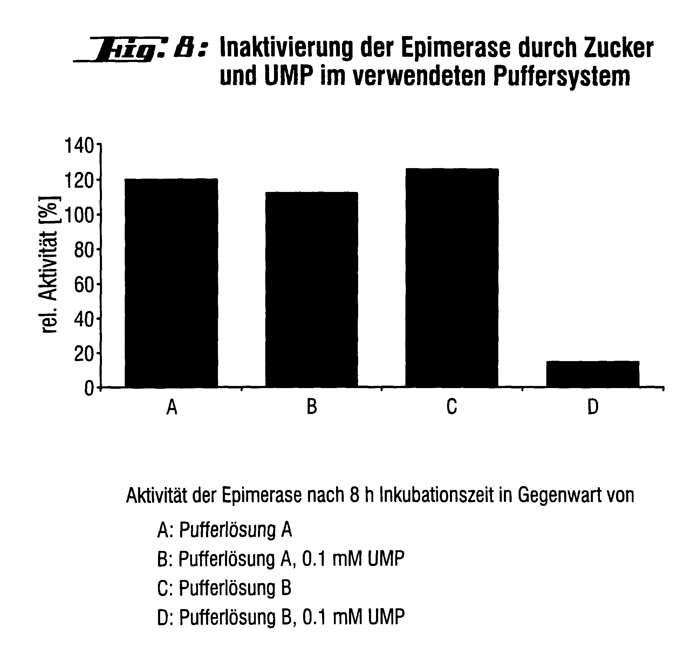

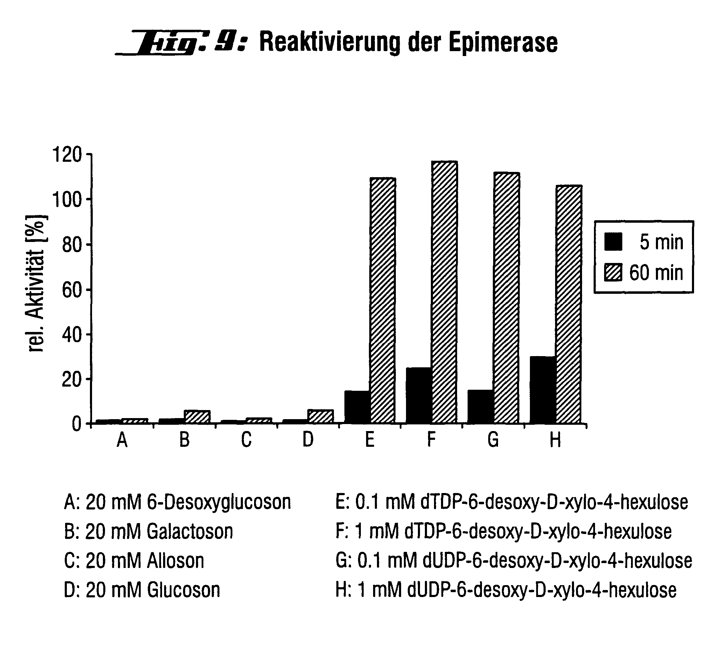

Das dritte Enzym im LacNAc-Zyklus nach Elling et al. (DE 42 21 595 C1) ist die UDP-Glucose-4'-Epimerase [E.C. 5.1.3.2.]. Das bei der Firma Sigma käufliche Enzym aus Saccharomyces cerevisiae besteht aus zwei Untereinheiten, an die ein Molekül NAD fest, aber nicht kovalent gebunden ist. (Fucusawa et al. J. Biol. Chem. 255, 2705 (1980)). Somit benötigt dieses Enzym keinen extern zugesetzten Cofaktor. Die Eigenschaften der E. coli und Hefe-Epimerasen wurden von Frey et al. beschrieben (in D. Dolphin et al (Eds.) Pyridine Nucleotide Coenzymes: Chemical, Biochemical and Medicinal Aspects, Vol. 2B, Wiley, New York, 462 - 511).
Die Epimerisierung erfolgt durch die Oxidation der UDP-Glucose zu UDP-4'-Ketopyranose und dessen anschließende Reduktion zum C-4'-Epimer des Eduktes (Fig. 4)
In Gegenwart von bestimmten Zuckern, in Anwesenheit von UMP oder UDP sowie durch die Substrate UDP-Glucose und UDP-Galactose kommt es zur reduktiven Inaktivierung der Epimerase (Carmenes et al, Yeast 2, 101 (1986), Nelestuen et al., J. Biol. Chem. 4, 7533 (1971)). Uridinnukleotide wie z. B. UMP induzieren durch ihre Bindung an das Enzym eine Konformationsänderung, bei der die Reaktivität von gebundenem NAD zu reduzierenden Substanzen erhöht ist. Die anschließend gebildete Epimerase-NADH zeigt nur noch 10 - 15 % der Aktivität des nativen Enzyms (Kalckar et al., Proc. Nat. Ac. Sci. 65, 1113 (1970)).

Wegen der Inaktivierung der Epimerase erreichen die bisher beschriebenen enzymatischen Synthesen von LacNAc mit in situ-Regenerierung der Nukleotidzucker nur geringe Zyklenzahlen und, besonders bei nicht natürlichen Substraten, nur unbefriedigende Ausbeuten. Will man die Ausbeuten an Di- oder Oligosaccharid erhöhen, ist ein mehrmaliges Nachdosieren der Epimerase unerläßlich, was die Synthese unwirtschaftlich macht.

Aufgabe der vorliegenden Erfindung ist es demgegenüber, ein wirtschaftliches Verfahren zur enzymatischen Synthese von Di- und Oligosacchariden bereitzustellen, bei welchem die Desaktivierung der UDP-Glucose-4'-Epimerase vermieden wird.

Die Aufgabe wird gelöst durch ein Verfahren zur enzymatischen Galactosylierung von Mono- und Oligosacchariden unter in situ Regeneration des Nukleotidzuckers in Gegenwart von Saccharose-Synthase, β-1-4-Galactosyltransferase und Uridindiphosphat-Glucose-4'-Epimerase, das dadurch gekennzeichnet ist, daß dem Reaktionsgemisch als Aktivator der Uridindiphosphat-Glucose-4'-Epimerase ein Ketozucker oder ein Ketozuckerderivat zugesetzt wird.

Als Aktivator eignet sich besonders ein Desoxynukleosiddiphosphat-Ketozucker, vorzugsweise Desoxyuridindiphosphat-6-desoxy-D-xylo-hexulose oder Desoxythymidindiphosphat-6-desoxy-D-xylo-hexulose.

Als Aktivator eignet sich auch ein Ketozucker. Vorzugsweise wird 6-Desoxyglucoson, Galactoson, Alloson oder Glucoson eingesetzt.

Bei dem erfindungsgemäßen Verfahren beträgt die Konzentration des Aktivators im Reaktionsgemisch 0,01 bis 20 mM, vorzugsweise 0,1 bis 1 mM.

Das Verfahren gemäß der vorliegenden Erfindung kann auch als Repetitive-Batch-Verfahren in einer Ultrafiltrationszelle ausgeführt werden.

Mit Hilfe des erfindungsgemäßen Verfahrens gelingt eine Reaktivierung der UDP-Glucose-4'-Epimerase, ohne daß die anderen Enzyme, Saccharose-Synthase und β-1-4-Galactosyltransferase, in ihrer Aktivität beeinträchtigt werden.

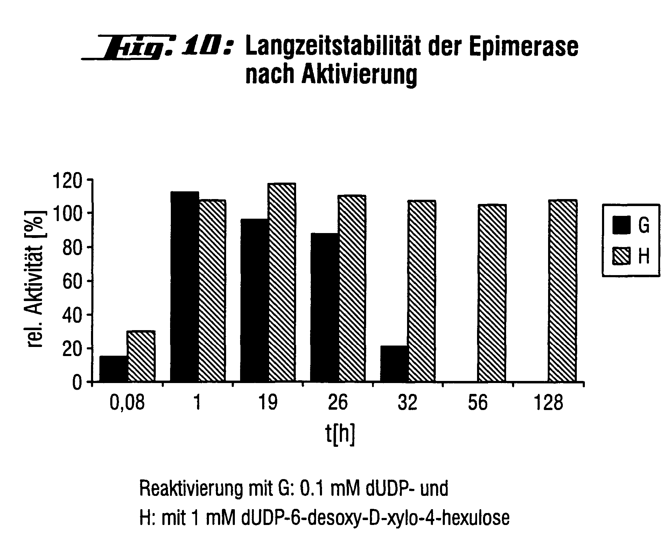

Bei Stabilitätsuntersuchungen der UDP-Glc-4'-Epimerase zeigte sich, daß die Epimerase aus Hefe in Gegenwart von UDP-Glucose und UDP-Galactose (Donor für die Galactosyltransferase) sowie in Gegenwart verschiedener Akzeptoren (Glc, 2-Desoxy-Glucose, 5-Thioglucose, n-Octylglucopyranosid) inaktiviert wird. Es handelt sich hier um ein Problem, das generell bei der Verwendung der Epimerase zur in situ Regenerierung von UDP-Galactose auftritt. Im Rahmen weiterer Versuche konnten wir nun zeigen daß insbesondere dUDP-6-Desoxy-D-xylo-4-hexulose sowie dTDP-6-Desoxy-D-xylo-4-hexulose zur Reaktivierung der UDP-Glc-4'-Epimerase eingesetzt werden können (Fig. 5).

dUDP- und dTDP-6-Desoxy-D-xylo-4-hexulose werden unter Katalyse der dTDP-Glucose-4,6- Dehydratase [EC 4.2.1.46] aus dUDP- und dTDP-Glucose gebildet (Zarkowsky et al. J. Biol. Chem. 244, 4750 (1969)).

dTDP-6-Desoxy-D- xylo-4-hexulose ist ein Zwischenprodukt auf dem Biosyntheseweg der dTDP-L-Rhamnose. Die enzymatische Synthese und Isolierung dieser Substanz wurde von Marumo et al. (Eur. J. Biochem. 204, 539 (1992)) beschrieben. dUDP-Glc ist nicht käuflich erhältlich, wurde jedoch von Melo et al. ( J. Biol. Chem. 240, 398 (1965)) mittels der dTDP-Glc-Pyrophosphorylase aus Pseudomonas aerugeriosa in analytischen Mengen synthetisiert. Unter Nutzung des Synthesepotentials der Saccharose Synthase können dUDP- und dTDP-6-Desoxy-D-xylo-4-hexulose ausgehend von dUMP bzw. dTDP hergestellt werden (Fig. 6).

dUDP-6-Desoxy-D-xylo-4-hexulose wurde anschließend erstmals zur Reaktivierung der Epimerase bei der Synthese von N-Acetyllactosamin (LacNAc) eingesetzt. Die Epimerase-Aktivität wurde über einen Zeitraum von 128 h verfolgt. Durch Zusatz von 1 mM dUDP-6-Desoxy-D-xylo-4-hexulose konnte eine schnelle und über den Beobachtungszeitraum stabile Aktivierung der Epimerase erreicht werden (Fig. 10).

Eine weitere Verbesserung des vorgeschlagenen Verfahres wird durch die Anwendung des Repetitive-Batch-Verfahrens (U. Kragl et al., Tetrahedron 4, 1193 - 1202 (1993)) ereicht. Die Umsetzung der Substrate erfolgt hierbei in Gegenwart von Saccharose-Synthase, Galactosyltransferase, Epimerase und dUDP-6-Desoxy-D-xylo-4-hexulose in einer Ultrafiltrationszelle mit einer YM10-Membran. Nach erfolgter Umsetzung wird die Produktlösung über die Ultrafiltrationsmembran abfiltriert, wobei die Enzyme zurückgehalten werden. Durch Zugabe frischer Substratlösung kann die Reaktion mehrmals wiederholt werden, ohne die Enzyme nachzudosieren. Dadurch können die nativen Enzyme ohne Immobilisierung wiederholt zur Synthese eingesetzt werden. Die Reaktivierung der Epimerase gewährleistet hierbei eine optimale Nutzung des Repetitive-Batch-Verfahrens zur wirtschaftlichen Synthese von LacNAc und dessen Analoga.

### Beispiele:

### Beispiel 1: Synthese von dUDP-6-Desoxy-D-xylo-4-hexulose ausgehend von dUDP-Glc

### Syntheseansatz:

- 20.1 mg: dUDPGlc (ca. 10 mM, siehe 1.2.4)
- 1920 *µ*l: Hepes-NaOH (200 mM, pH 7.2, 1 mM DTT, 500 mM Saccharose, 25 mM KCI, 1 mg/ml BSA)
- 80 *µ*l: dTDP-D-glc 4,6-dehydratase (1.48 U, Rohextrakt)

### Inkubation bei 30 °C, Inkubationszeit: 4 h

Nach 4 h konnte keine dUDP-Glc auf der HPLC mehr nachgewiesen werden (Fig. 9). Das Produkt wurde erfolgreich zur Reaktivierung der Epimerase eingesetzt. Die Synthese von dTDP-6-desoxy-D-xylo-4-hexulose erfolgte unter analogen Versuchsbedingungen. Hier konnte eine vollständige Umsetzung bereits nach 1 h beobachtet werden.

### Beispiel 2: Synthese von dUDP-6-Desoxy-D-xylo-6-hexulose ausgehend von dUMP

### Syntheseansatz:

### V = 3 ml

4mM dUMP (Na-Salz, Sigma® ), 4mM PEP (CHA-Salz, Biomol® ), 0.8 mM MgCl₂, 0.12 mM ATP (Na-Salz, Sigma® ), 500 mM Saccharose, 6 S.Syn. (2 U/ml), 60 U Pyruvatkinase (20 U/ml), 3 U NMPK (1 U/ml), 15 U dTDP-D-glc 4,6-dehydratase (5 U/ml),
- Puffer:: Tris-HCI (100 mM, pH 7.2, 3 mM DTT, 1 mg/ml Rinderserumalbumin (BSA), 50 mM KCI)
- Inkubationstemperatur:: 25 °C

Nach einer Inkubationszeit von 4 h konnte die Bildung von 69.3 % des Produktes beobachtet werden.

### Beispiel 3: LacNAc-Synthese nach dem Repetitive-Batch-Verfahren

Durch den Einsatz des repetitive Batch Verfahrens soll die Produktivität der Synthese deutlich gesteigert werden.

### Optimale LacNAc-Synthese:

1 mM UDP-Glc, 1 mM MnCl₂, 10 mM GlcNAc, 500 mM Saccharose, 0.05 U/ml GalT, 0.2 U/ml Epimerase, 0.4 U/ml Saccharose-Synthase, Puffer: 200 mM Hepes-NaOH, pH 7.2, 0.1 % BSA, 1 mM DTT
- Inkubationstemperatur:: 30 °C
- Umsatz:: 100 %
- Zyklenzahl:: 10
- Produktivität: (R-Z-A = Raum-Zeit-Ausbeute): 200 mM*ml/U R-Z-A : 3.8 g/I*d
- Syntheseansatz:: 1 ml des optimierten LacNAc-Ansatzes
Nach 12 Stunden bei 30 °C wurden ca. 750 *µ*l der Produktlösung mittels einer Zentricon® YM 10 abzentrifugiert.
Diafiltration der verbleibenden ca. 250 *µ*l mit Puffer ohne Rinderserumalbumin (BSA).

Die Enzymlösung wurde in Eppendorf® -Cups überführt und mit Substratlösung auf 1 ml aufgefüllt.
Die Probennahme erfolgt jeweils zu Beginn und nach Ende der Reaktion.

### Ergebnisse

Die Ergebnisse in Fig. 7 zeigen, daß die Epimerase nach 12 h Reaktionszeit und der Zentrifugation über die YM10-Membran nicht mehr aktiv war. Aufgrund dieses Ergebnisses wurde die Stabilität der Epimerase im eingesetzten Puffersystem genauer untersucht.

### Beispiel 4: Untersuchungen zur Epimerasestabilität Inaktivierung der Epimerase durch Zucker in Gegenwart von UMP

Die Epimerase wurde im verwendeten Puffersystem inkubiert und die Enzymaktivität über einen Zeitraum von 8 Stunden verfolgt.

### Versuchsbedingungen:

### Pufferlösungen:

A: 200 mM Hepes pH 7.2, 1 mM DTT, 1 mg/ml BSA
B: 200 mM Hepes pH 7.2, 1 mM DTT, 1 mg/ml BSA, 500 mM Saccharose Ansatz:
   A. Pufferlösung A
   B. Pufferlösung A, 0.1 mM UMP
   C. Pufferlösung B
   D. Pufferlösung B, 0.1 mM UMP
   mit jeweils 0.25 mg/ml Epimerase
   Inkubationstemperatur: 30 °C

Aktivitätstest: (Fukusawa et al., J. Biol. Chem. 255, 2705 - 2707 (1980))
- 893 *µ*l: 100 mM Glycin-Puffer pH 8.8
- 20 *µ*l: 5 mM UDP-Gal
- 20 *µ*l: 50 mM NAD
- 33,3 *µ*l: UDP-Glc-Dehydrogenase (2 U/ml)
- 33,3 *µ*l: Epimerase
Temperatur: 25 °C
Messung bei 340 nm

### Ergebnisse

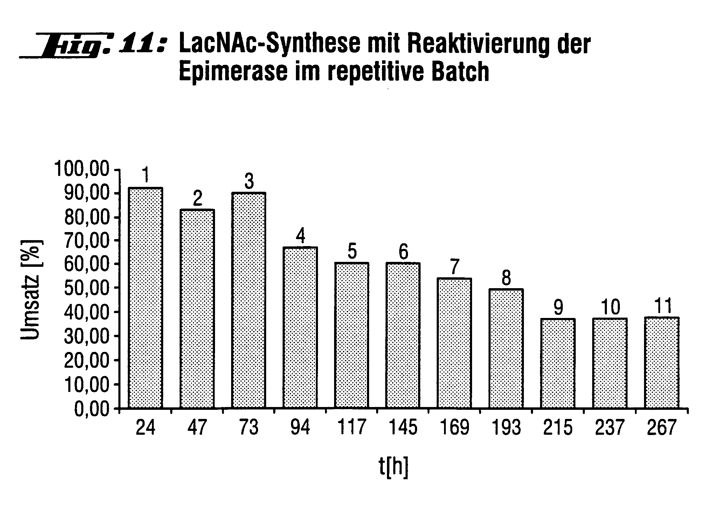

Die Figur 8 faßt die Ergebnisse zusammen und verdeutlicht, daß es in Anwesenheit von Saccharose bzw. ihrer Spaltprodukte, Glucose und Fructose, und UMP zur Inaktivierung der Epimerase kommt.

### Beispiel 5: Inaktivierung der Epimerase in Gegenwart verschiedener Akzeptoren und Donoren der GalT

Um die These, daß die Inaktivierung der Epimerase bei vielen Anwendungen auftreten kann, zu überprüfen, wurde die Stabilität der Epimerase in Gegenwart verschiedener Donoren und Akzeptoren der GalT getestet. Die Ergebnisse sind in der Tabelle 1 zusammengefaßt.

**Tab. 1:**

| Rel. Aktivität der Epimerase in Gegenwart verschiedener Donoren und Akzeptoren der GalT | | | | | |
|---|---|---|---|---|---|
| Inkubationszeit: | 2h | 7h | 8h | 24h | R |
| Donoren: | | | | | |
| P*, 1 mM UDP-Glc pH 7.2 | 80.7 | - | 80.7 | 58.3 | 137 |
| P*, 1 mM UDP-Glc pH 8.0 | 83.3 | - | 67.9 | 35.5 | 128 |

| Akzeptoren: | | | | | |
|---|---|---|---|---|---|
| P, 50 mM GlcNAc | 93.6 | 97.5 | - | - | - |
| P, 50 mM 2-Desoxyglc | 20.7 | - | - | - | - |
| P, 50 mM Glc | 15.8 | - | - | - | - |
| P, 50 mM Thioglc | 87.9 | 34.3 | - | - | - |
| P, 50 mM n-Octylglucopyranosid | 86.1 | 75.6 | 29.3 | - | - |
| P: 200 mM Hepes-NaOH, pH 7.2, 1 mM DTT, 1 mg/ml BSA, 25 mM KCI mit 0.1 mM UMP P*: Puffer ohne UMP R: Aktivität nach Reaktivierung der Epimerase mit dTDP-6-desoxy-D-xylo-4-hexulose (c = 0.1 mM) | | | | | |

Die Ergebnisse in der Tabelle 1 verdeutlichen, daß es in Gegenwart verschiedener Akzeptoren der GalT (Glc, Thioglc, 2-Desoxyglc, n-Octylglucopyranosid) in Gegenwart von UMP und UDP-Gal bzw. UDP-Glc zur Inaktivierung der Epimerase kommt. Somit handelt es sich hier um ein Problem, das generell bei der Verwendung der Epimerase zur in situ Regenerierung von UDP-Gal auftritt.

### Beispiel 6: Reaktivierung der Epimerase

Zur Reaktivierung der Epimerase wurden neben dUDP- und dTDP-6-desoxy-D-4-xylo-hexulose, 6-Desoxyglucoson, Galactoson, Alloson und Glucoson eingesetzt.

### Versuchsbedingungen:

### 1. Synthese von dUDP- und dTDP-6-desoxy-D-xylo-4-hexulose (siehe Beispiel 1)

### 2. Inaktivierung der Epimerase

- Inkubationsansatz:: 50 mM Galactose
0.1 mM UMP
0.25 mg/ml Epimerase
- Puffer:: 20 mM Hepes-NaOH pH 7.2
1 mM Dithiothreitol
1 mg/ml Rinderserumalbumin (BSA)
25 mM KCL

Nach einer Inkubationszeit von 2 h bei 30 °C wurde eine Aktivität der Epimerase von 3 % gemessen.

### 3. Aktivierung der Epimerase

- Inkubationsansatz:: 160 *µ*l inaktivierte Epimerase
40 *µ*l Aktivator (unterschiedliche Konzentrationen)

Die Ergebnisse der Aktivierung der Epimerase können Figur 9 entnommen werden. Die Epimerase-Aktivität wurde in den Proben G und H über einen Zeitraum von 128 h verfolgt. Die Ergebnisse sind in Figur 10 zusammengefaßt.

Die Ergebnisse zeigen, daß durch den Zusatz von dTDP- und dUDP-6-desoxy-D-xylo-4-hexulose eine schnelle und lange andauernde Aktivierung der Epimerase erreicht werden kann. Die Desaktivierungsgeschwindigkeit ist konzentrationsabhängig, so kann bei einer Konzentration von 0.1 mM bereits nach 32 Stunden eine erneute Inaktivierung der Epimerase aufgrund der noch im Ansatz vorhandenen Galactose und dem UMP beobachtet werden.

### Beispiel 7: LacNAc-Synthese im Repetitive Batch Verfahren mit Reaktivierung der Epimerase

### Material und Methoden

183 mg UDP-Glc (Na-Salz, Sigma® , 1 mM), 597 mg GlcNAc (10 mM), 46.2 mg MnCl₂ (1 mM), 46.2 g Saccharose (500 mM), 1.25 U GalT (0.05 U/ml), 5 U Epimerase (0.2 U/ml), 10 U Saccharose-Synthase (0.4 U/ml) und 25 mg BSA wurden in 200 mM Hepes-NaOH (1 mM DTT, 25 mM KCI, pH 7.2) zur LacNAc-Synthese eingesetzt. Das Reaktionsvolumen im Batch betrug zehnmal 25 ml und einmal 20 ml (Gesamtvolumen: 270 ml). Zur Reaktivierung der Epimerase wurden jeweils 250 *µ*l (Batchansatz 11 : 200 *µ* l) des Syntheseansatzes (Beispiel 1) (ca. 0.1 mM dUDP-6-desoxy-D-xylo-4-hexulose) zugesetzt. Die Diafiltration erfolgte in einer 50 ml Amicon® -Zelle mit einer YM10-Membran. Der Reaktionsansatz wurde dreimal auf ein Volumen von 50 ml mit Puffer aufgefüllt und auf 25 ml eingeengt. Bei der letzten Filtration wurde das Volumen auf 20 ml reduziert und die folgende Umsetzung durch Zugabe von 5 ml Substratlösung gestartet. Der Reaktionsansatz wurde nach Zugabe der Substratlösung steril filtriert. Das Filtrat wurde bei - 20 °C gelagert.
- Inkubationszeit:: 21 - 30 h
- Inkubationstemperatur:: 30 °C

Der Syntheseverlauf ist in Figur 11 dargestellt. In 11 Tagen wurden 597 mg GIcNAc (2.7 mmol) zu 594 mg LacNAc (1.55 mmol) umgesetzt, was einer mittleren Ausbeute von 57.4 % entspricht.

### Beispiel 8: Produktreinigung: LacNAc

### 1. Spaltung der Saccharose mit Invertase

* 25.000 U/ml Invertase (Sigma, Sacch. cerevisiae) in Puffer B (3.3.1) 2 h bei 45 °C vorinkubiert;
* Zugabe von 10 *µ*l Invertase / ml Produktlösung
* Inkubation bei 45 °C und regelmäßige Reaktionskontrolle über das Polarimeter
* Nach 120 Minuten wurde das Protein über eine YM10 Membran abgetrennt.

Das Filtrat wurde in 5 Chargen aufgeteilt.

### 2.) Zuckertrennung über die Calciumsäule

Säule: AG50W-X8 (5 x 35 cm) in Ca-Form, Elutionsmittel: bidest. Wasser, Fluß: 0.5 ml/min.

Die Probe wurde von der Probenaufgabe am Rotationsdampfer (20 - 25 mbar, 30 - 35 °C) auf ca. 30 ml aufkonzentriert.
Nach der Zuckertrennung wurden die Fraktionen, die LacNAc enthielten, gepoolt.

### 3.) lonenaustauschchromatographie an Dowex® 1 x 2 Cl^{⊖} (100 - 200 mesh)

Säule: 2.6 x 26.5 cm, Fluß, 3.5 ml/min, Elutionsmittel: bidest. Wasser Der pH-Wert der Probe wurde auf 8.5 eingestellt. Die Fraktionen, die LacNAc enthielten, wurde gepoolt und der pH-Wert auf 7.0 eingestellt.

### 4.) Gelfiltration mit der P₂-Säule

Säule: Biorad® -P₂-Säule (2.6 x 82 cm), Elutionsmittel: bidest. Wasser, Fluß: 0.5 ml/min.

Die Fraktionen, die LacNAc enthielten, wurden gepoolt.

Die Chargen 1 - 5 wurden vereinigt.

### 5.) Die noch vorhandenen Reste an Hepes wurden durch eine erneute lonenaustauschchromatographie (siehe 3.) abgetrennt.

### 6.) Das Produkt wurde mittels zweier Gelfiltrationsläufe weitgehend entsalzt (siehe 4.)

### 7.) Zur Erhöhung der Produktreinheit trennte man einen Teil des noch vorhandenen GlcNAcs und Salzes mittels einer Ca-Säule (siehe 2.) ab.

### 8.) Die Produktlösung wurde am Rotationsverdampfer aufkonzentriert und anschließend gefriergetrocknet.

- Auswaage nach Synthese:: 594 mg
- Auswaage nach Reinigung:: 356 mg (59.9 %)

### Beispiel 9: Synthesen mit verschiedenen Akzeptoren und Donoren der GalT

Im Rahmen weiterer Synthesen wurden bisher folgende Donoren und Akzeptoren im LacNAc-Zyklus eingesetzt:
- Donoren:: dUDP-Glc
- Akzeptoren:: 2-Desoxy-D-glucose, 5-Thio-D-glucose, Glc, n-Octylglucopyranosid, n-Octylthioglucopyranosid, 6-Aminohexyl-N-acetylglucosaminid.

### Variation des Donors

### Versuchsbedingungen:

optimierter LacNAc-Ansatz mit 0.05 U/ml GalT mit Reaktivierung der Epimerase mit 1 mM dUDP Glc.
Analytik: HPLC-Methode LacNAc

### Ergebnisse:

Bei der Synthese mit 0.05 U/ml GalT wurden 12.5 % LacNAc nach 17 Stunden gebildet.

### Synthese von Lactoseanaloga

### Versuchsbedingungen:

- Syntheseansatz:: optimierter Syntheseansatz mit 0.1 mM dTDP-6-desoxy-D-xylo-4-hexulose und 0.1 mg/ml α-Lactalbumin
- Akzeptoren:: 2-Desoxyglc, Thioglc, Glc, n-Octylglucopyranosid, n-Octylthioglucopyranosid
- Blindprobe:: Syntheseansatz ohne Akzeptor
HPLC-Analytik für 2-Desoxyglc, Thioglc, Glc
- Säule:: Aminex HPX-87C bei 85 °C, Fluß: 0,5 ml/min
- Laufmittel:: bidest. Wasser, Detektor: a) UV-Vis 205 nm
b) Chiralyzer

DC-Analytik für n-Octylglucopyranosid, n-Octylglucopyranosid, 6-Aminohexyl-N-Acetyl-glucosaminid
- Laufmittel:: 85 : 12 : 3 (n-Propanol : HAC : H₂O)
- Sprühmittel:: 50 % methanolische H₂SO₄

### Ergebnisse:

Die Ergebnisse zeigten, daß die Bildung von Lactose bei allen Synthesen stattgefunden hat. Bei der Synthese mit Glc als Akzeptor wird deutlich mehr Lactose als bei den anderen Synthesen gebildet. Thioglucose, n-Octylthioglucopyranosid und n-Octylglucopyranosid werden zu den jeweiligen Disacchariden umgesetzt, während die Umsetzung von 2-Desoxyglc zunächst nicht nachgewiesen werden kann.

### Beispiel 11: Präparative Synthesen

### 1. Präparative Synthese von N-Octyl-4-β-D-Galactopyranosyl-D-glucopyranosid und 4-O-β-Galactopyranosyl-D-2-desoxyglucose

41 mg UDP-Glc (Na-Salz, Sigma® , 1 mM), 175 mg n-Octylglucopyranosid (Südzucker, 10 mM) bzw. 97 mg 2-Desoxyglc (Fluka® , 10 mM), 12 mg MnCl₂ (1 mM), 10.3 Saccharose (500 mM), 1.2 GalT (0.06 U/ml), 4 U Epimerase (0.2 U/ml), 8 U Saccharose-Synthase (0.4 U/ml), 6 mg Lactalbumin (0.1 mg/ml) und 20 mg BSA wurden in 200 mM Hepes-NaOH (1 mM DTT, 25 mM KCI, pH 7.2) zur Synthese eingesetzt. Das Reaktionsvolumen betrug dreimal 20 ml (Gesamtvolumen: 60 ml). Zur Reaktivierung der Epimerase wurden täglich 200 *µ*l des Syntheseansatzes (Beispiel 1) ca. 0.1 mM dTDP-6-desoxy-D-xylo-4-hexulose zugesetzt. Die Diafiltration erfolgte in einer 50 ml Amicon® -Zelle mit ein YM10-Membran.
- Inkubationszeit:: 2 Tage pro Ansatz
- Inkubationstemperatur:: 30 °C

### Produktreinigung:

### 1. N-Octyl-4-β-D-Galactopyranosyl-D-glucopyranosid

Die Produktlösung wurde fraktionsweise (5 Fraktionen) über 5 Sep-Pack C-18 Reverse-Phase-Säulen von Waters® (Mississauga, Ont., Canada) (Palcic et al., Glycoconjugate J. 5, 49 - 63 (1988)) gegeben. Vorbereitung der Säulen: Spülen mit 10 ml Methanol und 20 ml bidest. Wasser, Probeaufgabe, Spülen der Säule mit 20 ml bidest. Wasser, Elution des Produkts und Edukts mit 10 ml Methanol.

Das Methanol wurde am Rotationsverdampfer bei 30 °C und 120 mbar abgezogen und die Zucker in bidest. Wasser aufgenommen. Die Trennung der Dissaccharids vom Monosaccharid erfolgte über eine P2-Säule (2.6 x 82 cm,
- Fluß:: 0.5 ml/min, bidest. Wasser)
- Auswaage:: 58.3 mg, Ausbeute: 21.4 %.

### 2. 4-O-β-Galactopyranosyl-D-2-desoxyglucose

Die Produktreinigung erfolgte in Analogie zur Reinigung von LacNAc. Es wurde die für die N-Octyl-4-β-D-Galactopyranosyl-D-glucopyranosid beschriebenen DC-Methode zur Analytik eingesetzt. (Auswaage: 53.4 mg ≙ 28.7 %.)

## Patentansprüche

1. Verfahren zur enzymatischen Galactosylierung von Mono- und Oligosacchariden unter in situ Regeneration des Nukleotidzuckers in Gegenwart von Saccharose-Synthase, β-1-4-Galactosyltransferase und Uridindiphosphat-Glucose-4'-Epimerase, **dadurch gekennzeichnet, daß** dem Reaktionsgemisch als Aktivator der Uridindiphosphat-Glucose-4'-Epimerase ein Ketozucker oder ein Ketozuckerderivat zugesetzt wird.

2. Verfahren nach Anspruch 1, **dadurch gekennzeichnet, daß** als Aktivator ein Desoxynukleosiddiphosphat-Ketozucker eingesetzt wird.

3. Verfahren nach Anspruch 2, **dadurch gekennzeichnet, daß** als Aktivator Desoxyuridindiphosphat-6-desoxy-D-xylo-hexulose eingesetzt wird.

4. Verfahren nach Anspruch 2, **dadurch gekennzeichnet, daß** als Aktivator Desoxythymidindiphosphat-6-desoxy-D-xylo-hexulose eingesetzt wird.

5. Verfahren nach Anspruch 1, **dadurch gekennzeichnet, daß** als Aktivator ein Ketozucker eingesetzt wird.

6. Verfahren nach Anspruch 5, **dadurch gekennzeichnet, daß** als Aktivator 6-Desoxyglucoson eingesetzt wird.

7. Verfahren nach Anspruch 5, **dadurch gekennzeichnet, daß** als Aktivator Galactoson eingesetzt wird.

8. Verfahren nach Anspruch 5, **dadurch gekennzeichnet, daß** als Aktivator Alloson eingesetzt wird.

9. Verfahren nach Anspruch 5, **dadurch gekennzeichnet, daß** als Aktivator Glucoson eingesetzt wird.

10. Verfahren nach einem der Ansprüche 1 bis 9, **dadurch gekennzeichnet, daß** die Konzentration des Aktivators im Reaktionsgemisch 0,01 bis 20 mM beträgt.

11. Verfahren nach Anspruch 10, **dadurch gekennzeichnet, daß** die Konzentration des Aktivators im Reaktionsgemisch 0,1 bis 1 mM beträgt.

12. Verfahren nach einem der Ansprüche 1 bis 11, **dadurch gekennzeichnet, daß** das Verfahren als Repetitive-Batch-Verfahren in einer Ultrafiltrationszelle durchgeführt wird.

## Claims

1. A process for enzymically galactosylating monosaccharides and oligosaccharides, with in-situ regeneration of the nucleotide sugar, in the presence of sucrose synthase, β-1-4-galactosyl transferase and uridine diphosphate-glucose 4'-epimerase, wherein a ketosugar or a ketosugar derivative is added to the reaction mixture as an activator of the uridine diphosphate-glucose 4'-epimerase.

2. The process as claimed in claim 1, wherein a deoxynucleoside diphosphate ketosugar is employed as the activator.

3. The process as claimed in claim 2, wherein deoxyuridine diphosphate-6-deoxy-D-xylohexulose is employed as the activator.

4. The process as claimed in claim 2, wherein deoxythymidine diphosphate-6-deoxy-D-xylohexulose is employed as the activator.

5. The process as claimed in claim 1, wherein a ketosugar is employed as the activator.

6. The process as claimed in claim 5, wherein 6-deoxyglucosone is employed as the activator.

7. The process as claimed in claim 5, wherein galactosone is employed as the activator.

8. The process as claimed in claim 5, wherein allosone is employed as the activator.

9. The process as claimed in claim 5, wherein glucosone is employed as the activator.

10. The process as claimed in one of claims 1 to 9, wherein the concentration of the activator in the reaction mixture is from 0.01 to 20 mM.

11. The process as claimed in claim 10, wherein the concentration of the activator in the reaction mixture is from 0.1 to 1 mM.

12. The process as claimed in one of claims 1 to 11, wherein the process is carried out as a repetitive-batch process in an ultrafiltration cell.

## Revendications

1. Procédé de galactosylation enzymatique de mono- et d'oligosaccharides avec régénération in situ du sucre nucléotidique en présence de saccharose-synthase, de β-1,4-galactosyltransférase et d'uridinediphosphate-glucose-4'-épimérase, **caractérisé en ce qu'**on ajoute au mélange réactionnel, en tant qu'activateur de l'uridinediphosphate-glucose-4'-épimérase, un cétosucre ou un dérivé de cétosucre.

2. Procédé selon la revendication 1, **caractérisé en ce qu'**on utilise en tant qu'activateur un désoxynucléoside-diphosphate-cétosucre.

3. Procédé selon la revendication 2, **caractérisé en ce qu'**on utilise en tant qu'activateur du désoxyuridine-diphosphate-6-désoxy-D-xylo-hexulose.

4. Procédé selon la revendication 2, **caractérisé en ce qu'**on utilise en tant qu'activateur du désoxythymidine-diphosphate-6-désoxy-D-xylo-hexulose.

5. Procédé selon la revendication 1, **caractérisé en ce qu'**on utilise en tant qu'activateur un cétosucre.

6. Procédé selon la revendication 5, **caractérisé en ce qu'**on utilise en tant qu'activateur de la 6-désoxyglucosone.

7. Procédé selon la revendication 5, **caractérisé en ce qu'**on utilise en tant qu'activateur de la galactosone.

8. Procédé selon la revendication 5, **caractérisé en ce qu'**on utilise en tant qu'activateur de l'allosone.

9. Procédé selon la revendication 5, **caractérisé en ce qu'**on utilise en tant qu'activateur de la glucosone.

10. Procédé selon l'une des revendications 1 à 9, **caractérisé en ce que** la concentration de l'activateur dans le mélange réactionnel est de 0,01 à 20 mM.

11. Procédé selon la revendication 10, **caractérisé en ce que** la concentration de l'activateur dans le mélange réactionnel est de 0,1 à 1 mM.

12. Procédé selon l'une des revendications 1 à 11, **caractérisé en ce que** le procédé est mis en oeuvre sous forme d'un procédé discontinu répétitif dans une cellule d'ultrafiltration.
